# EUROPEAN PATENT APPLICATION

(11) **EP 4 023 770 A1**
(43) Date of publication of application: **06.07.2022**
(21) Application number: 21460001.7
(22) Date of filing: 05.01.2021
(51) Int. Cl.: C12Q 1/6886

(54) **A METHOD OF EXAMINING GENES FOR THE DIAGNOSIS OF THYROID TUMORS, A SET FOR THE DIAGNOSIS OF THYROID TUMORS AND APPLICATION**

(71) Applicant: Narodowy Instytut Onkologii im. Marii Sklodowskiej-Curie Panstwowy Instytut Oddzial w Gliwicach, 44-102 Gliwice (PL); Politechnika Slaska, 44-100 Gliwice (PL); WASKO Spólka Akcyjna (WASKO S.A.), 44-100 Gliwice (PL)
(72) Inventor: Oczko-Wojciechowska, Malgorzata, 41-808 Zabrze (PL); Kotecka-Blicharz, Agnieszka, 44-100 Gliwice (PL); Krajewska, Jolanta, 44-100 Gliwice (PL); Wilk, Agata, 44-100 Gliwice (PL); Jarzab, Michal, 41-807 Zabrze (PL); Fujarewicz, Krzysztof, 43-100 Tychy (PL); Jarzab, Barbara, 44-101 Gliwice (PL); Student, Sebastian, 44-100 Gliwice (PL); Pfeifer, Aleksandra, 41-705 Ruda Slaska (PL); Kowalska, Malgorzata, 44-100 Gliwice (PL); Tyszkiewicz, Tomasz, 44-100 Gliwice (PL); Pluciennik, Alicja, 44-100 Gliwice (PL); Stobiecka, Ewa, 44-100 Gliwice (PL)

(57) **Abstract**

A method of testing genes for diagnosing thyroid nodules in an unspecified sample from biopsy (FNAB) material, from which RNA is isolated, then RNA upon transcription is synthesized into cDNA, followed by determination and normalization of gene expression. The material is a frozen or a freshly collected residual biopsy (washout needle) from washing the residue in the bevel of the needle, containing RNA at a concentration ranging from 4 ng/µl to 20 ng/µl, which is pre-amplified before transcription of total RNA at a number of cycles depending on RNA concentration. The length of the obtained gene amplicons ranges between 53base pairs and 134 base pairs. In addition, the expression of genes selected from transcription factors of the corresponding target gene is analyzed, and the normalization of expression of target genes is carried out under endogenous control of at least four normalizing genes, preferably six normalizing genes. The use of genes from the set including C3, CAMK2N1, DUSP5, EGR1, EMP2, FAM20A, FAXC, ITGA2, KCNQ3, MET, METTL7B, MPZL2, PSD3, SDC4, SLC26A4, SLPI, TM4SF1, TPO, SLC26A7, and TUSC3, for diagnosing of thyroid nodules in an ultra-small residual biopsy sample or full biopsy with RNA concentrations ranging from 4 ng/µl to 20 ng/µl. (10 claims)

## Description

The invention relates to and can be used for preoperative differential diagnosis of benign and malignant thyroid nodules. The ultra-small residual amount of material from a fine-needle aspiration biopsy (FNAB) of a nodule is used for diagnosis. To implement the present invention, a set of genes suitable for supporting the diagnosis of thyroid cancer in an ultra-small residual amount of FNAB material is described.

Differential diagnosis of thyroid nodules is essential for determining indications for thyroid surgery. The current diagnostic methods do not provide complete safety in assessing that we are dealing with a benign thyroid nodule, which can be left without surgery without any harm.

The normal follicular thyroid cell is highly differentiated, with specialized features including the ability to respond to thyrotropic hormone (TSH), iodine transporters, the ability to bind iodine with thyroid peroxidase (TPO) and store it bounded to thyroglobulin (Tg), and to maintain the follicular structure. Abnormal proliferation of thyroid cells can result in the formation of nodules, which may be benign or malignant. Thyroid malignancies come in three main forms: papillary, follicular, and anaplastic carcinoma. Medullary thyroid carcinoma (MTC) originates from the parafollicular C cells.

Papillary thyroid carcinoma (PTC) is the most common type of differentiated thyroid carcinoma originating from the follicular cell. The greatest challenge is preoperative diagnosis of follicular thyroid carcinoma (FTC). These cancers - papillary and follicular - are collectively referred to as differentiated thyroid carcinomas (DTC). Routine diagnosis of suspected thyroid nodules is based on ultrasound imaging and cytological evaluation of material (biological sample) obtained by fine-needle aspiration biopsy and sometimes other percutaneous and surgical biopsy techniques.

The material (biological sample) includes tissue samples taken from the tumor by puncture and/or aspiration, spread on a microscope slide for analysis, or preserved as a suspension in fixative fluid for further processing. Part of the same FNAB sample fresh frozen or after storage in frozen form as well as samples from residual material (needle washout) left in the dead space (bevel) of the biopsy needle are used for the analysis. However, samples with insufficient or low-quality nucleic acids shall be excluded from the analysis.

Currently, the result of a cytological examination is used to determine further clinical management. When the result suggests a suspicion of a malignant lesion, surgical treatment is required, whereas when a benign lesion is identified, follow-up is usually applied. The Bethesda System for Reporting Thyroid Cytology (TBSRTC) is used to determine the risk of malignancy of the examined cells in a cytological smear, which is based on the classification of the cytological picture in 6 categories from non-diagnostic lesions - I to malignant neoplasm - VI, where category III is undefined lesion or atypia of undetermined significance (AUS) or follicular lesion of undetermined significance (FLUS), and a category IV lesion is a follicular neoplasm or suspicious for a follicular neoplasm.

FNAB is a standard diagnostic procedure for suspected thyroid nodules. However, it has many limitations due to its inability to assess the arrangement of follicular cells within the thyroid follicle and to evaluate any invasive features. The amount of material obtained from FNAB is small. Thus, the use of immunohistochemical methods or other support of classical pathological diagnostic techniques is difficult. For this reason, nearly 30% of all biopsies of thyroid nodules are classified as indeterminate lesions, while only 15-30% of them are malignant lesions that require surgical intervention. It means that most of the nodules removed are benign and do not require such a radical medical procedure. So, thyroid surgery may be considered unnecessary.

Supportive testing for thyroid cytology, including widespread molecular diagnostics, could reduce the number of diagnostic surgeries resulting in a better quality of life for patients and less burden on the health care system. However, it is unclear to what extent gene-level analysis can approximate diagnostic estimates, as it may not capture the full differences between the genomes of malignant and benign thyroid nodules. At US2007037186A1, for example, Jiang et al. disclosed a 4-gene RT-PCR assay with the sensitivity of a cancer diagnosis of 92%, but a specificity of only 61%.

Studies on molecular alterations that play a role in the development of thyroid cancer revealed that the point mutation of the BRAF gene T1799A leading to the exchange of amino acids V600E within the BRAF protein and constitutive activation of serine/threonine kinase activity, observed in about 45% of PTCs, is the most frequent. RAS mutations, also involved in the formation of thyroid tumors, are frequently found in follicular adenomas, suggesting their potential role in early oncogenesis in the follicular cell. Mutations in PTEN, PIK3CA, and other genes, and the translocations (RET-PTC, PAX8- PPARG) or abnormal gene methylation, are considered important. However, the use of some of these molecular markers in preoperative diagnostics is still insufficient to distinguish benign from malignant tumors with adequate sensitivity and specificity.

There are known gene classifiers based on the evaluation of mutations of several or even dozens of genes or assessing the expression of a set of genes. The most common is the expression classifier resulting from the study by Chudova *et al.,* where the test (gene expression classifier, GEC) is based on assessing the expression of a set of mRNAs using high-density oligonucleotide microarrays. The test is divided into several stages, and for the lesion considered suspicious, an additional assessment of the BRAF gene mutations and RET/PTC1 and RET/PTC3 rearrangements is performed. It is a "rule out malignancy" test, and its result serves primarily to exclude the malignancy of the nodule. The Afirma Gene Expression Classifier (Veracyte Inc, USA) was developed based on the above-mentioned study.

In the Genomic Sequencing Classifier (GSC), based on next-generation sequencing (NGS), both gene expression and mutations are analyzed. The test allows the analysis of several thousand genes, including the detection of changes originating from Hürthle cells.

There are also known tests based solely on the analysis of mutations in the tumor (DNA) and similar ones with an additional analysis of microRNA (miRNA) expression to achieve higher sensitivity and specificity.

However, there is a lack of no widely conducted validation tests involving the European population for the gene classifiers mentioned above.

The method of RNA analytics, known from the description of the patent EP3119886B1, is a method preserving copy number used for RNA testing to estimate the transcript amount and type. A method for generating nucleic acid product from a template RNA molecule comprising after having obtained a template RNA: annealing the first oligonucleotide primer at a preselected nucleic acid region of the template RNA, elongating the first oligonucleotide primer in a template-specific manner, thereby obtaining the first elongated strand, removing the RNA template, annealing more than one further oligonucleotide primers to the first elongated strand, elongating more than one further oligonucleotide primers in a template-specific manner without displacement of primers annealed to the first elongated strand using a polymerase lacking strand displacement activity, isolating and/or amplifying an elongation product comprising a nucleic acid portion that is elongated complementary to the first oligonucleotide primer. The invention also relates to the use of a kit performing a method.

The invention, known from the description of the patent EP2505664B1, is devoted to distinguishing between malignant and benign thyroid tumors by performing RNA extraction in a standard FNAB sample, in particular in an air-dried FNAB smear. The isolated RNA is analyzed for the presence of gene rearrangements and/or miRNA expression, whereby the presence of RET/PTC and/or PAX8/PPARG rearrangements and/or mutations in genes encoding BRAF, N-, K- and/or HRAS and/or differential miRNA expression in the sample classifies the tumor as malignant.

The use of differentially expressed genes in benign and malignant thyroid lesions for diagnosis and staging of thyroid cancer is disclosed in the description of the patent EP1756303B1. According to the patent US8202692B2, the level of expression of kallikrein 10 in thyroid tissue is detected. An increased level of kallikrein 10 expression is indicative of thyroid cancer. While to provide a diagnosis, the patent US8541170B2 relates to compositions, kits, and methods for molecular profiling and cancer diagnostics, including gene expression product markers, alternative exon usage markers, and DNA polymorphisms associated with cancer. Similarly, the description of the patent US7901888B2 provides methods for diagnosing, prognosis, and staging in thyroid cancer using molecular markers. Methods for identifying compounds useful in the treatment or prevention of thyroid cancer are also disclosed.

The system for classifying thyroid nodule as malignant or benign, known from the description of the patent US9617604B2, is based on identifying sets of gene transcripts. It involves the analysis of an expression level of one or more transcripts in a sample from a thyroid nodule, at least one of which includes one transcript corresponding to at least one sequence selected from SEQ ID No. 1-12, 261, 283-306, 657, 658, and 659. Further, the classification system provides sets of target sequences and combinations of polynucleotide probes and primers prepared in solution or in the matrix.

The patents US10236078B2 and US10672504B2 concern the compositions and methods for molecular profiling and diagnostics of genetic disorders and cancer, including markers of gene expression products associated with cancer or genetic disorders. The invention provides algorithms and methods of classifying thyroid cancer, methods of determining molecular profiles, and methods of analyzing results to make a diagnosis. To obtain a data set on gene expression, gene expression products in a tissue sample are evaluated by sequencing, array hybridization, or nucleic acid amplification.

The invention US10260103B2 concerns diagnostic assays for identifying thyroid cancer in a biological sample, including FNAB aspirate. It also provides related compositions and kits useful in applying the methods of the invention. Moreover, it discloses the method of thyroid cancer diagnosing by determining an expression level of three or more gene products in a thyroid tissue sample, the gene products consisting of gene products expressed by the CXCR3, CCR3, CXCL10, CAR, XB130, HO-1, and CCR7 genes.

The invention known from the description of the patent US10422009B2 provides a system for classifying thyroid nodule as malignant or benign based on identifying sets of transcripts. The thyroid classification system provides for sets of thyroid classifying target sequences and further provides for combinations of polynucleotide probes and primers derived there from. These combinations of probes and arrays can be used for diagnosis. In the tested sample, the first group of transcripts includes at least two transcripts having at least 90% sequence homology to at least two sequences selected from SEQ ID No. 1-6, 11-13, 16-248. The second group of transcripts in the tested sample includes at least two transcripts showing at least 90% sequence homology to at least two sequences selected from SEQ ID No. 7-10, 14, 15, 249-584.

Diagnosing a genetic disorder or cancer, as known from the invention US20202974A1 comprises classifying a biological sample by introducing one or more differential levels of gene expression products into a trained algorithm, wherein technical factor variables are removed from data based on differential gene expression product level and normalized prior and during classification. The trained algorithm classifies a sample as positive for a genetic disorder or cancer at a given level of specificity and/or sensitivity.

The patent EP2569626B 1 describes the compositions, kits, and methods of molecular profiling for diagnosing thyroid conditions. The method for evaluating a thyroid tissue sample is based on determining an expression level for one or more gene expression products correspond to at least 5 genes selected from SNCA, STK32A, THRSP, TIMP1, TIMP2, TMSB10, TNFRSF 17, TNFRSF1A, TXNDC12, VWA5A, WAS, WIPI1, and ZFYVE16 and subsequent classifying of the thyroid tissue sample by comparing expression levels to gene expression data for at least two different sets of biomarkers. The gene expression data for each set of biomarkers includes one or more reference gene expression levels correlated with the presence of one or more tissue types. The expression level is compared sequentially for at least two different sets of biomarkers.

However, according to the patent EP2925886B1, a thyroid sample is identified as malignant or cancerous if the expression level of any one or more of CXCR3, CXCL11, SPAG-9, CAR, Nectin-1, XB-130 and/or CXCL4 genes is decreased; and/or the expression of one or more of **C**XCR3A, CXCR3B, CXCR4, CCR3, CXCL9, CXCL 10, CK-19, TIMP-1, CLDN-1 and/or CCR7 is increased in the thyroid sample as compared to the normal control expression level. The total number of genes with increased or decreased expression is at least three.

The following terms are used in the description and claims of the patent and its summary:
The **"thyroid nodule"** is defined as any focal lesion in the thyroid gland, benign or malignant, which can be subjected to a diagnostic process for the definitive assessment of nature and clinical aggressiveness
**gene expression** - the process by which the genetic information contained in a gene is read and transcribed into its products, which are proteins or different forms of RNA;
**evaluation of gene expression** - intended to mean the determination of the amount or presence of a mRNA transcript or the product of expression of a specific gene;
**gene expression profiling -** is the analysis of the expression of the set of genes
**gene expression profile** - means both the whole set of information concerning a set of genes and the information regarding their expression, both considered as a whole and as a selected part, identifiable for a given type of tissue, cell, or their function;
**gene expression classifier** (GEC) - a set of genes selected to determine the characteristics or condition of the cells under study and to classify the results;
**reference gene,** control gene, normalizing gene - a gene whose expression is maintained at a constant level in cells, regardless of their origin and phenotype - both in normal and neoplastic cells;
**residual (aspiration) biopsy, residual biopsy or washouts** - a sample obtained by rinsing out the biopsy needle after making a cytological preparation, containing DNA and RNA;
**frozen biopsy -** includes specimens fixed in paraffin, stored in liquid nitrogen or dry ice, as well as specimens fixed with fixatives, etc.;
**PPV (positive predictive value)** - the probability that an individual has had the disease with a positive test result. So, if a person obtained a positive test result, the PPV indicates how confident they can be that they have the disease (based on the Clopper-Pearson method);
**NPV (negative predictive value)** - the probability that an individual does not have the disease with a negative test result. So, if a tested person received a negative test result, the NPV indicates the probability that an individual does not suffer from a given disease;
**ROC (Receiver Operating Characteristic Curve)** is a tool to assess the quality of a classifier. I∼zt provides a combined description of its sensitivity and specificity;
**TPR - True Positive Rate;**
**FPR - False Positive Rate False;**
**QPCR (Quantitative Real-time polymerase chain reaction)** Real-time polymerase chain reaction to evaluate transcript expression
**DNA microarray -** a plate with applied probes allowing the assessment of expression of many thousands of transcripts simultaneously, approximately determines the expression of the entire transcriptome of a given cell

### SUMMARY OF THE INVENTION

The present disclosure of the invention concerns to a method of testing genes for the diagnosis of thyroid tumors in the material of an unspecified FNAB sample of a thyroid nodule. The sample (a residual biopsy; needle washout) is obtained by washing out with buffer the residue in the dead space (bevel) of the needle, containing RNA at concentrations ranging from 4 ng/µl to 20 ng/µl.

The expression level and use of a set of genes involving C3, CAMK2N1, DUSP5, EGR1, EMP2, FAM20A, FAXC, ITGA2, KCNQ3, MET, METTL7B, MPZL2, PSD3, SDC4, SLC26A4, SLPI, TM4SF1, TPO, SLC26A7, and TUSC3 while combined expression levels above certain threshold point a malignant character of the nodule.

In another set of genes, the expression of 7 genes such as CAMK2N, EGR1, EMP2, ITGA2, KCNQ3, TM4SF1, and TPO is evaluated.

The present disclosure also concerns a kit to diagnose thyroid nodules in a biopsy sample, which includes a device with at least one processor and at least one memory program with processor execution instructions for analyzing data and determining the gene expression by the classifier algorithm. Furthermore, it includes the equipment, as well as appropriate reagents, solutions, probes, and primers for reverse transcription reaction and devices with equipment for detecting the expression of a set of 20 genes or at least 7 genes from the set, including C3, CAMK2N1, DUSP5, EGR1, EMP2, FAM20A, FAXC, ITGA2, KCNQ3, MET, METTL7B, MPZL2, PSD3, SDC4, SLC26A4, SLPI, TM4SF1, TPO, SLC26A7, and TUSC3.

A small amount of FNAB material remaining in the needle after the biopsy is used for the test, while the remaining material can be used for other necessary tests, which significantly increase the reliability of the test and reduces its inconvenience for the patient, who can avoid multiple thyroid biopsies and reduces the cost of the test.

### FIGURES

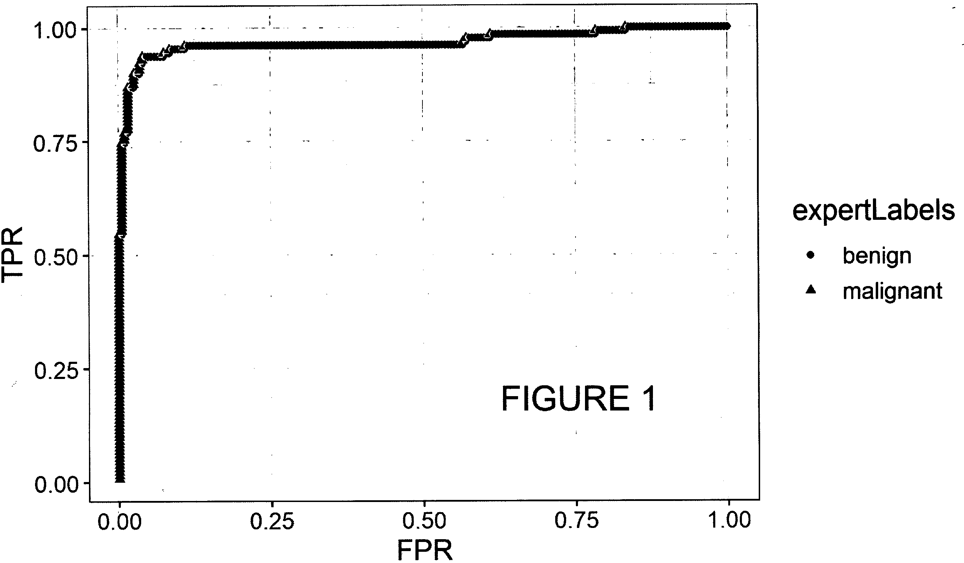

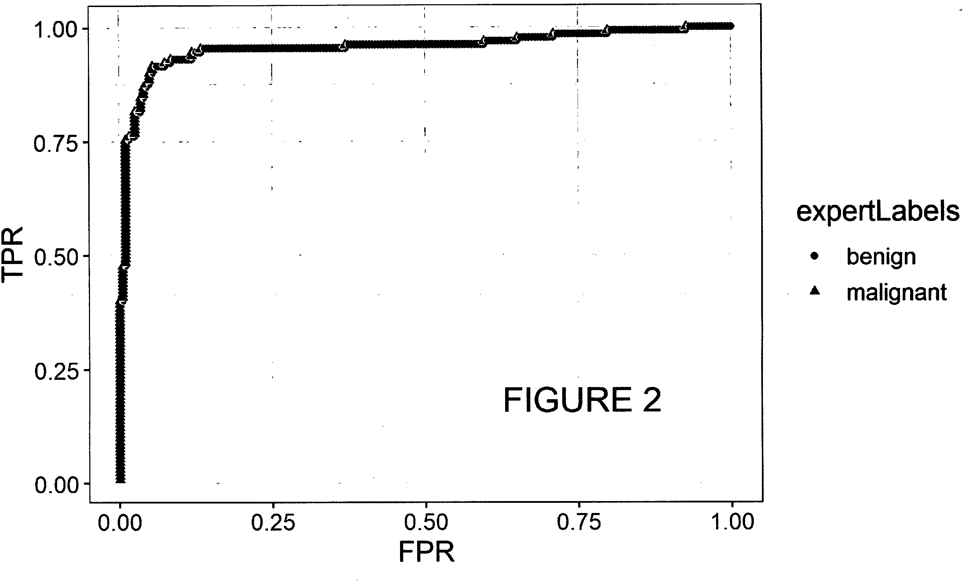

Figure 1 and Figure 2 show diagrams that illustrate the selection of the probability cut-off threshold for a 20-gene classifier and a 7-gene classifier, respectively.

In each graph, the TPR (Y-axis) expresses the sensitivity of the classifier, as a ratio of the number of observations correctly classified positive cases (detection of thyroid cancer) to all observations correctly identified by the classifier and is a number in the range 0-1.

The FPR (X-axis) expresses the ratio of all incorrectly classified positive cases (thyroid cancer marked as a benign lesion) to the number of all observations marked as negative (benign lesion) by the classifier. It is calculated as specificity, and the number is in the range 0-1. The area under the ROC curve shows the characteristics of sensitivity and specificity of the classifier. For the 20-gene classifier and 7 gene classifier for both graphs, the ROC curve reaches values close to 1 for low values on the X-axis, indicating the good performance of classifiers that correctly label the observations.

Figure 3 shows the graph of quality dependence on the number of features for the FoldChange selection method.

Figure 4 and Figure 5 show a plot of the dependence of the cut-off threshold by controlling the sensitivity and test specificity, including PPV and NPV parameters.

Figure 6, Figure 7, Figure 8, and Figure 9 illustrate in box and mandolin plots the use of 20-gene and 7-gene classifiers to predict tumor malignancy.

### DETAILED DISCLOSURE OF THE INVENTION

In the diagnostics of a thyroid nodule, ultrasound is the first imaging step after clinical evaluation. Depending on the estimated risk for a given image, taking into account tumor diameter and other parameters, the indications for further evaluation of the thyroid nodule are defined. Fine needle aspiration biopsy (FNAB) of a thyroid nodule provides material for cytological analysis, the result of which brings clinically useful data. If the result falls into one of the undetermined categories, i.e., Bethesda III (B3), IV (B4), or V (B5) is the subject of additional investigation.

Diagnosing a thyroid tumor as benign or malignant by classifying it based on gene expression values requires a testing procedure and multidimensional analysis of FNAB tumor sample, containing material for RNA isolation, and an expression classifier.

More than 3,000 FNAB samples of thyroid tumors were collected to identify genes for the diagnostics of thyroid nodules using the test kit, as well as in the earlier research phase to build the classifier. One or two additional tumor samples were taken from each patient, to obtain an adequate amount of biological material to isolate nucleic acids for the molecular analysis.

The study used total RNA isolated from FNAB material of thyroid nodules. Material collected by aspiration is preserved in preservation buffer, frozen, and then, following RNA isolation (alone or together with DNA isolation at the same time), gene expression evaluation by QPCR, microarrays, NGS, or other methods. Based on the clinical parameters of the nodule, cytological data, and gene expression data a molecular test is carried out.

In addition, each of the above 3000 tumor samples has a pair, which is a residual biopsy (washout needle) made by rinsing the biopsy needle with an appropriate buffer after the cytological preparation. In practice, the needle after making a cytological preparation is discarded, whereas, in the project, the needle was used to obtain residual biopsy material. Needles with biopsy material were rinsed in a solution of RLT buffer and beta-mercaptoethanol and then stored in a freezer at -20°C. At the same time, not every sample from a full biopsy contains complete material useful for obtaining RNA in the amount necessary for studies according to current standards.

Within the framework of IT for supporting medical diagnosis in oncology, including thyroid cancer, the data on the results of tumor sample analyses are additionally combined with information from cytological, molecular, and other data such as tumor grade and stage and comorbidities, graphical and textual medical imaging data, in order to obtain reliable data for disease prediction. High-throughput molecular tests, including, but not limited to, protein mass spectrometry, quantitative PCR, microarrays, or sequencing, provide meaningful diagnostic data. Information on thyroid cancer includes age, sex, lesion size, shape, Bethesda categories, tumor multifocality, echogenicity, additional information on affected lymph nodes.

Furthermore, the framework of IT supporting medical diagnosis includes an expressive classifier system (hereinafter classifier) with at least one processor and at least one program memory, which contains the execution instructions of the processor (algorithm classifier) for analyzing data and determining gene expression levels. The classifier contains test data sets of full biopsy specimens as described above and linked by patient ID datasets from residual biopsies of thyroid nodules, with different training sets (1) and (2) taking into account the specificity of the input data.

The expression classifier was built based on a Support Vector Machine (SVM) model with a radial kernel function, trained on measured gene expressions for patient samples of the training set. All values are estimated using a Bayesian clinical reference model to standardize parameters, discover and include examples i.e., members of the input set representative of the clusters. The inclusion of the prediction of clinical malignancy risk in the thyroid nodule as a highly relevant synthetic feature is significant. Each sample is identified by a four-element vector (four probabilities): malignancy risk (RofM), taking into account patient sex, age, nodule size, and Bethesda score. The classifier returns a prediction for thyroid nodule samples for one of two classes: benign lesion and malignant (malignant neoplasm), and depending on the need, the nature of the test can be a cancer exclusion test or a cancer confirmation test. The classifier was trained from expression measurements performed by RT-QPCR on a population of training samples containing material from full-thickness and residual biopsies.

To compare the quality of the classifiers based on the selected gene set - training series and validation was conducted on several subsets. Each of the training sets, i.e., training set (1) and training set (2), contains different proportions of residual biopsy samples. The training set (1) consisted of 348 FNAB samples subjected to gene expression analysis based on transcriptome (mRNA) assessment using microarrays. The training set (2) consisted of 130 samples selected among above 3,000 residual biopsies that were subjected to gene expression analysis based on transcriptome (mRNA) assessment by microarray studies using Human Gene 2.0 microarrays.

In total, 872 samples were used to build and validate the classifier, including 269 residual biopsies and 603 full FNAB samples, obtained from RT-QPCR analysis (genes selected based on microarray analysis). Most of the residual biopsy samples have a corresponding full biopsy obtained from the same patient.

To prevent information leakage from the training set to the test set, the set contains a single sample from a given patient. Biopsies for which there are residual biopsy equivalents in the learning set are excluded from the collection.

The samples excluded from the training set created an additional set, the partially dependent validation set, Validation P1U. The remaining samples were divided into three validation sets, as follows:

| **collection** | **description** | **Number of samples** | **washout number** | **number of tumor samples** |
|---|---|---|---|---|
| **Training** | training set, samples numbered up to G4324, excluding biopsy samples that have a washout equivalent in the collection | 333 | 119 | 130 |
| **Validation_P** | validation set, washout samples with numbers above G4324 (washings corresponding to the Validation_P1V set) | 150 | 150 | 96 |
| **Validation_P1** | Validation collection, biopsies numbered above G4324 that do not have a washout pair | 136 | 0 | 57 |
| **Validation_P1U** | Validation set, biopsies numbered up to G4324 having a washout pair in the learning set | 103 | 0 | 42 |
| **Validation_P1V** | Validation set, biopsies numbered above G4324 that have a washout pair in the validation set (biopsies corresponding to Validation_P set) | 150 | 0 | 96 |
| **Validation** | Validation_P + Validation_P1 | 286 | 150 | 153 |

### Classification quality - for a 20-gene classifier:

| **Validation set** | **accuracy** | **sensitivity** | **specificity** | **PPV** | **NPV** |
|---|---|---|---|---|---|
| **Validation** | 0.864 | 0.791 | 0.947 | 0.945 | 0.797 |
| **Validation_P** | 0.860 | 0.792 | 0.981 | 0.987 | 0.726 |
| **Validation_P1** | 0.868 | 0.789 | 0.924 | 0.882 | 0.859 |
| **Validation_P1U** | 0.893 | 0.833 | 0.934 | 0.897 | 0.891 |
| **Validation_P1V** | 0.867 | 0.823 | 0.944 | 0.963 | 0.750 |
| **Validation - only Bethesda III, IV and V** | 0.865 | 0.763 | 0.955 | 0.937 | 0.822 |
| **Validation_P - only Bethesda III,IV and V** | 0.866 | 0.776 | 0.981 | 0.981 | 0.773 |
| **Validation_P1 - only Bethesda III, IV and V** | 0.865 | 0.733 | 0.932 | 0.846 | 0.873 |
| **Validation_P1U - only Bethesda III, IV and V** | 0.932 | 0.889 | 0.957 | 0.923 | 0.936 |
| **Validation_P1V - only Bethesda III, IV and V** | 0.857 | 0.791 | 0.942 | 0.946 | 0.778 |

### Classification quality - for the 7-gene classifier:

| **Validation set** | **accuracy** | **sensitivity** | **specificity** | **PPV** | **NPV** |
|---|---|---|---|---|---|
| **Validation** | 0.853 | 0.765 | 0.955 | 0.951 | 0.779 |
| **Validation_P** | 0.827 | 0.740 | 0.981 | 0.986 | 0.679 |
| **Validation_P1** | 0.882 | 0.807 | 0.937 | 0.902 | 0.871 |
| **Validation_P1U** | 0.903 | 0.859 | 0.934 | 0.900 | 0.905 |
| **Validation_P1V** | 0.833 | 0.771 | 0.944 | 0.961 | 0.699 |
| **Validation - only Bethesda III, IV and V** | 0.841 | 0.701 | 0.964 | 0.944 | 0.787 |
| **Validation_P - only Bethesda III,IV and V** | 0.815 | 0.687 | 0.981 | 0.978 | 0.708 |
| **Validation_P1 - only Bethesda III, IV and V** | 0.876 | 0.733 | 0.949 | 0.880 | 0.875 |
| **Validation_P1U - only Bethesda III, IV and V** | 0.918 | 0.889 | 0.935 | 0.889 | 0.935 |
| **Validation_P1V - only Bethesda III, IV and V** | 0.832 | 0.746 | 0.942 | 0.943 | 0.742 |

The microarray collection, after pre-processing and background correction of the entire microarray set, was normalized. A custom Chip Definition File (CDF) version 21 was used to define the probe sets representing genes from the ENTREZ database for all microarrays, which was downloaded from the website: http://brainarray.mbni.med.umich.edu/Brainarray/Database/CustomCDF/CDFdownload.asp

For variants and combinations of feature selection methods, data fusion, and classification algorithms, a study of the classification quality of feature models was conducted using the bootstrap method, where samples were drawn from the learning set (1The selection and classification algorithms with the best classification quality parameters were selected and based on them, by the resubstitution method (using a combination of selection algorithms on the full training set), a set of 20 genes was selected for the RT-QPCR plate - Open Array - according to Table 1.

**Table 1.**

| Gene Symbol(s) | Gene Name(s) | Gene Alia(es) | RefSeq(s) | GenBank. mRNA(s) | Amplicon. Length | sequence.amplicon |
|---|---|---|---|---|---|---|
| C3 | complement component 3 | AHUS5; ARMD9; ASP; C3a; C3b; CPAMD1; HEL-S-62p | NM_000064.3 | K02765.1; BC150179.1; EU794602.1; BC150200.1; BP201387.1 | 88 | ctgtgtggcagaccccttcgaggtcaca gtaatgcaggacttcttcatcgacctgcgg ctaccctactctgttgttcgaaacgagcag |
| C3a peptide modulates inflammation and exhibits antimicrobial activity. Mutations in this gene are associated with atypical hemolytic uremic syndrome and age-related macular degeneration in humans. | | | | | | |
| CAMK2N1 | calcium/calmodulin dependent protein kinase ll inhibitor 1 | PRO1489 | NM_018584.5 | AY204901.1 | 63 | cgggttgttattgaagatgatagg attgatgacgtgctgaaaaa tatgaccgacaaggcacct |
| CAMK2N1 is an endogenous inhibitor of CAMKII, a ubiquitous serine/threonine protein kinase that phosphorylates nearly 40 different proteins in response to intracellular calcium oscillations. | | | | | | |
| DUSP5 | dual specificity phosphatase 5 | DUSP; HVH3 | NM_004419.3 | BC062545.1; U16996.1 | 107 | aggggggatatgagactttctactcggaatat cctgagtgttgcgtggatgtaaaacccattt cacaagagaagattgagagt gagagagccctcatcagccagtgtg |
| The protein encoded by this gene belongs to the protein phosphatase subfamily with dual specificity. These phosphatases inactivate target kinases by dephosphorylating both phosphoserine/threonine and phosphotyrosine residues. Negatively regulate a superfamily of mitogen-activated protein (MAP) kinases (MAPK/ ERK, SAPK / JNK, p38) that are associated with cell proliferation and differentiation. This protein inactivates ERK1and is expressed in a variety of tissues with the highest levels of expression in the pancreas and brain. | | | | | | |
| EGR1 | early growth response 1 | AT225; G0S30; KROX-24; NGFI-A; TIS8; ZIF-268; ZNF225 | NM_001964.2 | KM114058.1; AK298101.1; X52541.1; M62829.1; BC073983.1; AK301065.1; M80583.1 | 72 | cttttcctgacatctctctgaacaac gagaaggtgctggtggagacca gttaccccagccaaaccactcgac |
| GR proteins. It is a nuclear protein and acts as a transcriThe protein encoded by this gene belongs to the C2H2 family of zinc finger Eptional regulator. It is a nuclear protein and acts as a transcriptional regulator. The products of the target genes it activates are involved in the processes of differentiation and mitogenesis. Studies suggest that it is a tumor suppressor gene. | | | | | | |
| EMP2 | epithelial membrane protein 2 | XMP | NM_001424.5; XM_006720864.3 | U52100.1; AK313134.1; BF981157.1; BC009687.1; X94770.1 | 88 | agagtactccacgctgcaggcggtcca ggccaccatgatcctctccaccattctct gctgcatcgccttcttcatcttcgtgctccag |
| This gene encodes a protein of the PMP22 / EMP family. The encoded protein regulates cell membrane composition. It is associated with a variety of functions, including endocytosis, cell signaling, cell proliferation, cell migration, cell adhesion, cell death, cholesterol homeostasis, urinary albumin excretion, and embryo implantation. It is known to negatively regulate Kaveolin-1, a scaffolding protein that is a major component of the cavities in the membrane of kaveoli found in most cell types. By activating PTK2, it positively regulates vascular endothelial growth factor A. It also modulates the function of specific integrin isomers in the cell membrane. Up-regulation of this gene has been linked to neoplastic progression in a wide variety of tissues. Mutations in this gene are associated with nephrotic syndrome type 10 (NPHS10). | | | | | | |
| FAM20A | family with sequence similarity 20 member A | Al1G; AIGFS; FP2747 | NM_001243746.1; XM_011524918.2; XM_006721959.2; XM_017024781.1; NM_017565.3 | BC136686.1; AL133105.1; AB545606.1; AK298071,1; AK056789.1; AY358197.1; BC136689.1 | 67 | aggcactttcccaccatcagtgctgatt acagtcaagatgagaaagccttgct gggggcatgtgact |
| This gene encodes a protein that is believed to play a role in hematopoiesis. Mutation at this locus is associated with amelogenesis imperfecta (congenital underdevelopment of enamel) and gingival hypertrophy syndrome. | | | | | | |
| FAXC | failed axon connections homolog | C6orf168; dJ273F20 | XM_006715582.2; XM_011536188.2; NM_032511.2 | BC011583.1; AK057793.1; BC004869.2; AK096480.1; BC006515.2 | 70 | caaaggtgagctgatcaaccttgccatg tactgtgagaggataaggaggaaatttt ggccagagtggcac |
| The gene encodes a protein that localizes to the nuclear membrane and belongs to the glutathione transferase (GST) family. In eukaryotes, GSt 13 are involved in the detoxification of reactive electrophilic compounds by catalyzing their coupling to glutathione. The GST domain is also found in proteins with unknown GST activity, such as eukaryotic elongation factor 1-gamma and the HSP26 family of stress-related proteins, which include auxin-regulated proteins. May play a role in axon development. | | | | | | |
| ITGA2 | integrin subunit alpha 2 | BR; CD49B; GPIa; HPA-5; VLA-2; VLAA2 | NM_002203.3; NR_073103.1; NR_073104.1; NR_073105.1; NR_073106.1 | BC143509.1; AK307952.1; BC143508.1; BP280760.1; X17033.1; BC143505.1; BC143511.1 | 67 | attttaaattgttgtttggcctacaa tgttggtctcccagaagcaaaaat attttccggtccttcaa |
| The gene encodes the alpha subunit of the trans-membrane receptor for collagen and related proteins. The encoded protein forms a heterodimer with the beta subunit and mediates adhesion of platelets and other cell types to the extracellular matrix. Loss of the encoded protein is associated with platelet-type bleeding disorder 9. Antibodies to this protein are found in various immune system disorders, including autoimmune thrombocytopenia in neonates. | | | | | | |
| KCNQ3 | potassium voltage gated channel subfamily Q member 3 | BFNC2; EBN2; KV7.3 | XM_006716555.3; XM_017013400.1; NM_004519.3; NM_001204824.1; XM_005250914.3; XM_011517026.2 | AK296293.1; AF033347.1; BC128576.1 | 55 | aaagaactcatcacggcctggtacatcgg tttcctgacactcatcctttcttcat |
| The gene encodes a protein that functions in regulating neuronal excitability. The encoded protein forms the M channel by association with the products of the related genes KCNQ2 or KCNQ5, which encode integral membrane proteins. Defects in this gene cause benign familial neonatal seizures type 2 (BFNC2). | | | | | | |
| METTL7B | methyltransferase like 7B | ALDI | DI NM_152637.2 | AY358508.1; AK290112.1 | 82 | agaccgggaggtgtgctctttttct gggagcatgtggcagaaccatat ggaagctgggccttcatgtggcag caagttttcg |
| It encodes an S-adenosyl-L-methionine-dependent methyltransferase family protein and a methyltransferase-like protein 7B. METTL7B plays a role in lipid metabolism, development of severe preeclampsia, and tumor progression. Previous studies have indicated that METTL7B may have a dual function in tumor progression. | | | | | | |
| MPZL2 | myelin protein zero like 2 | EVA; EVA1 | NM_144765.2; NM_005797.3 | AY359061.1; BC017774.1; AK290326.1; DA163069.1; AF275945.1; AF030455.1; AF304447.1 | 82 | ctctttggcctatagcagctgtggaaattt atacctcccgggtgctggaggctgttaat gggacagatgctcggttaaaatg |
| The gene is expressed in the thymus and several epithelial structures early in embryogenesis. Its ability to mediate cell adhesion through homophilic interaction and its selective regulation by T-cell maturation may suggest a role in the early stages of thymic organogenesis. The protein has a characteristic V-type domain and two potential N-glycosylation sites in the extracellular domain; a putative serine phosphorylation site for casein kinase 2 is also present in the cytoplasmic tail. | | | | | | |
| MET | MET protooncogene, receptor tyrosine kinase | AUTS9; DFNB97; HGFR; RCCP2; c-Met | XM_006715990.2; NM_001127500.2; NM_001324401.1; NM_001324402.1; XM_011516223.1; NM_000245.3 | EU826572.1; EU826573.1; EU826575.1; AB209898.1; EU826574.1; X54559.1; EU826576.1; J02958.1; EU176015.1; EU826567.1; BC130420.1; EU826577.1; EU826568.1; EU826569.1; EU826570.1; EU826579.1; EU826571.1; AK296974.1 | 73 | ggttgtggtttctcgatcaggacc atcaacccctatgtgaattttctcct ggactcccatccagtgtctcca |
| MET is a protooncogene encoding a membrane receptor with tyrosine kinase activity that is a hepatocyte growth factor receptor. The protein plays a role in cell survival, embryogenesis, and cell migration and invasion. Mutations in this gene are associated with papillary renal cell carcinoma, hepatocellular carcinoma head and neck cancers. Amplification and overexpression of this gene are also associated with other cancers. | | | | | | |
| PSD3 | pleckstrin and Sec7 domain containing 3 | EFA6D; EFA6R; HCA67 | XM_011544468.2; NM_015310.3; XM_011544473.2; XM_017013265.1; XM_011544469.2; XM_017013264.1; XM_011544467.2; XM_017013263.1; NM_206909.2; XM_011544471.2; XM_017013258.1; XM_017013262.1; XM_017013261.1; XM_017013260.1; XM_017013259.1 | GU727648.1; AK315099.1; AK091317.1; DB055312.1; AF243495.2; AF519767.1; AL832778.1; BC075045.2; BC075044.2; AB023159.2 | 83 | caggtatttctttaaagcattctctcttgt gggagaaactcaagaacgagagagag ttttaatacacttctccaatagatatt |
| The function of the encoded protein is not fully understood. PSD3 expression has been detected in pancreas, heart, muscle, and adipocytes. PSD3 is associated with immune diseases such as systemic sclerosis. Postulated as a suppressor gene in ovarian cancers. | | | | | | |
| 5DC4 | syndecan 4 | SYND4 | NM_002999.3; XM_011528977.2 | CR542074.1; X67016.1; BC030805.1; AK223243.1; AK303964.1; AK222695.1; D13292.1; AK312507.1; CR542045.1 | 147 | tggtgcctctagataaccatatccctg agagggcagggtctgggagccaag tccccaccgaacccaagaaactaga ggagaatgaggttatccccaa gagaatctcacccgttgaagagag tgaggatgtgtccaacaaggtgtcaa |
| The protein encoded by this gene is a trans-membrane (type I) proteoglycan (heparan sulfate) that acts as a receptor in intracellular signaling. | | | | | | |
| SLC26A4 | solute carrier family 26 member 4 | DFNB4; EVA; PDS; TDH2B | XM_005250425.2; XM_006716025.3; NM_000441.1; XM_017012318.1 | AF030880.1 | 72 | aagggatggcatatgccctactagct gcagttcctgtcggatatggtctct actctgcttttttccctatcc |
| The gene encodes the protein pendrin; an anion transporter. Mutations in this gene are associated with Pendred syndrome, an autosomal recessive disease. | | | | | | |
| SLC26A7 | solute carrier family 26 member 7 | SUT2 | NM_052832.3; NM_001282357.1; NM_001282356.1; NM_134266.1 | AJ413229.2; AF331521.1; BC114474.1; AJ413228.1; AJ413230.1; BC113866.1; AK122933.1 | 85 | gttgaggtttacatggactgtaaagg caggagtgtggatgtattgttagccca ttgtacagcttccttgataaaagcaatgacgt |
| A gene belonging to the sulfate/anion transporter gene family. It shows high expression in the kidney. | | | | | | |
| SLPI | secretory leukocyte peptidase inhibitor | ALK1;ALP; BLPI; HUSI; HUSI-I; MPI; WAP4; WFDC4 | NM_003064.3 | X04470.1; AF114471.1; AX772818.1; AK312192.1; BC020708.1; X04503.1 | 69 | ttcaaagctggagtctgtcctcctaag aaatctgcccagtgccttagatacaa gaaacctgagtgccag |
| A gene encoding a protease inhibitor that protects epithelial tissues from serine proteases. It is found in various secretions, including seminal plasma, cervical mucus, and bronchial secretions, and has affinity for trypsin, leukocyte elastase, and cathepsin G. Its inhibitory activity contributes to the immune response by protecting epithelial surfaces from attack by endogenous proteolytic enzymes. The protein has antibacterial, antifungal, and antiviral activity. | | | | | | |

The second variant of the gene set contains a selection of genes that includes the seven (7) genes presented in Table 2.

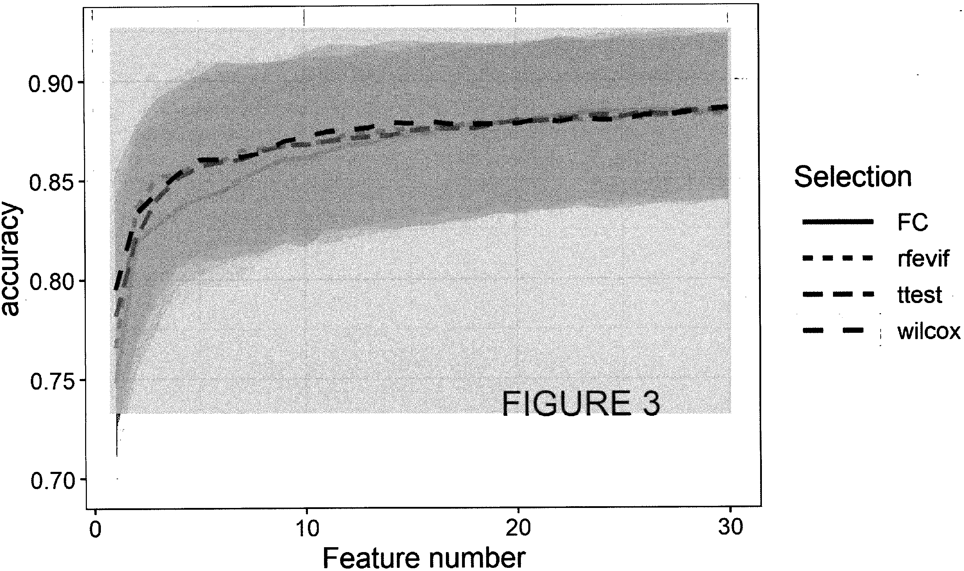

The number of genes meeting the quality criteria and the fewest possible features were selected for the classifier. An inflection point was observed for the quality plot for the FoldChange selection method, the median classification quality (accuracy) was 0.9, CI (0.79, 0.97) (Fig. 3).

The second gene set for the classifier selected based on resubstitution on the training set includes the following genes CAMK2N1, EGRI, EMP2, ITGA2, KCNQ3, TM4SF1, and TPO, summarized in Table 2.

**Table 2**

| No. | Gene name | ENTREZ ID | Assay ID | function |
|---|---|---|---|---|
| 1 | CAMK2N1 | 55450 | Hs00218591_m1 | Gene tested |
| 2 | EGR1 | 1958 | Hs00152928_m1 | Gene tested |
| 3 | EMP2 | 3013 | Hs00171315_m1 | Gene tested |
| 4 | ITGA2 | 3673 | Hs00158127_m1 | Gene tested |
| 5 | KCNQ3 | 3786 | Hs01120412_m1 | Gene tested |
| 6 | TM4SF1 | 4071 | Hs01547334_m1 | Gene tested |
| 7 | TPO | 7173 | Hs00892519_m1 | Gene tested |

The selection of reference genes was made by iterative exclusion of the least stable genes based on the NormFinder function. For this purpose, microarrays were selected from the training set and, 5 iterations of the algorithm were carried out. The list of the obtained most stable reference genes is shown in Table 3.

**Table 3.**

| No. | Symbol | ENTREZ | Assay_ID | |
|---|---|---|---|---|
| 1 | EIF3A (EIF3S10) | 8661 | Hs01025769_m1 | Check gene |
| 2 | HADHA | 30307322 | Hs00426191_m1 | Check gene |
| 3 | UBE2D2 | 7322 | Hs00366152_m1 | Check gene |
| 4 | EIFS | 1983 | Hs01028813_g1 | Check gene |
| 5 | PPIE | 10450 | Hs01102333_m1 | Check gene |
| 6 | RPLPO | 6175 | Hs00420895_gH | Check gene |

Selecting the performance of a classifier whose algorithms are based on the expression profile of genes from a given set of classifiers requires selecting the type of algorithm by specifying the corresponding set of genes, (based on the FoldChange algorithm) and then selecting the features according to the parametric Student's t-test.

In the first version of the classifier, a set of 20 the following genes were used C3, CAMK2N1, DUSP5, EGR1, EMP2, FAM20A, FAXC, ITGA2, KCNQ3, MET, METTL7B, MPZL2, PSD3, SDC4, SLC26A4, SLPI, TM4SF1, TPO, SLC26A7, TUSC3. The data are shown in Table 1.

In the second version, a classifier consists of a set of 7 genes: CAMK2N1, EGR1, EMP2, ITGA2, KCNQ3, TM4SF1, TPO, summarized in Table 2.

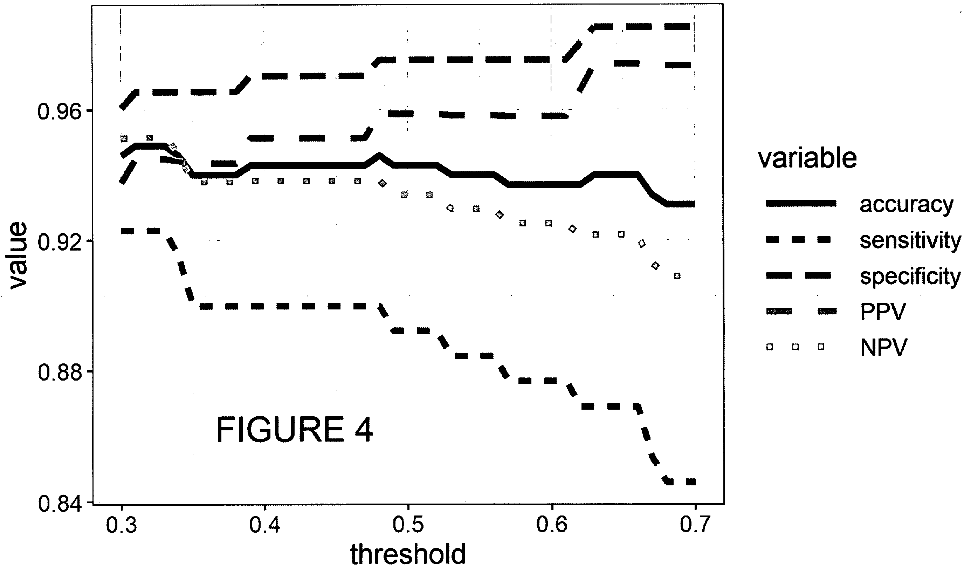

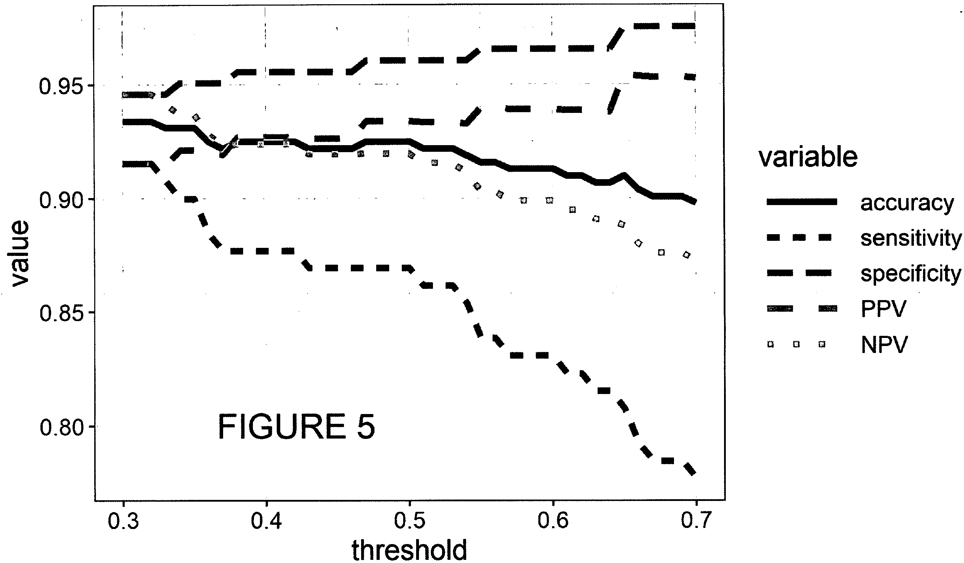

The cut-off threshold applied on the learning set based on cross-sectional empirical analyses enables control of the sensitivity and specificity of the test, including the PPV (positive predictive value) and NPV (negative predictive value) parameters, as shown in fig. 4 and fig. 5 for the 20-gene and 7-gene classifier, respectively. A threshold of 0.34 (34%) was set for the 20-gene classifier, for which the curves corresponding to positive and negative predictive value and accuracy intersect.
In the example performance, the classifier is optimized against PPV, and a high positive predictive value (PPV) confirms the malignancy of the cancer in the sample. The probability cut-off selection shown in fig.1 illustrates the effect of changing the probability above which a sample is considered malignant on the quality parameters for the 20-gene classifier. In contrast, fig. 2 shows this relationship for the 7th gen classifier.

The classification test includes a sample normalization step for the entire plate, and reference gene selection is performed using the GENORM method.

In contrast, normalization is performed using the BioMaps application and proprietary software, which takes into account the low expression signal during the assay for the calibrator samples, the lack of sufficient expression signal for the reference genes in the sample, and an overall assessment of the expression levels of the normalized genes of all test samples.
The classifier displays on the screen of the BioMaps application computer the result of the classification test with information about the prediction of the class, the probability of malignancy, the numerical values of all the features of the sample on the basis of which the classification took place. In addition, box plot or mandolin plot with points or histograms for the benign and malignant classes of each gene with the expression value of the test sample plotted, with the ability to print the result for the patient.

The performance of the classifier was confirmed under experimental conditions, where samples of thyroid nodules with an established malignant result were validated in the postoperative examination of resected thyroid nodule, whereas regarding benign samples by long-term follow-up.

The use of samples with a small amount of analyzed material for diagnostic testing has been validated in experimental studies. Samples collected by laser microdissection were used for this purpose, of which 18 contained papillary thyroid carcinoma and 15 samples contained normal thyrocytes. In addition, 15 complete sections were taken from the tumor, and 12 sections were taken from the normal thyroid gland. Gene expression evaluation was performed using microarrays HGU133.2.0 PLUS. The study showed that 15 thyroid follicles allowed for isolation of a sufficient amount of genetic material for molecular analysis and evaluation of the entire cell transcriptome. The suitability of residual biopsy material was verified on a training set (2), and 130 paired samples were subjected to whole transcriptome analysis using HTA-2.0 microarrays.

RNA isolation was performed using the RNeasy Micro Kit. The obtained RNA concentration of the RNA was measured with a microfluorometer, whereas a qualitative RNA assessment was performed using a bioanalyzer and RNA 6000 Nano Assay Kit. The average amount of RNA in the material from a complete tumor slice was 180.62 ng/µl. The mean RNA concentration in stained PTC and normal thyroid tissue slides were 5.05 ng/µl and 9.12 ng/µl, respectively. The mean RNA concentration in microdissected PTC cells was 5.57 ng/µl and in normal thyrocytes was 7.25 ng/µl. The amount of RNA obtained from full biopsies is higher than in residual biopsies. Nevertheless, the residual biopsy provided sufficient material to evaluate the gene expression profile by DNA microarray. The median concentration of total RNA obtained from the residual biopsies was 10.2 ng/µl, while the median concentration from the full tumor specimen was 14.545 ng/µl.

Examination of the thyroid transcriptome of papillary thyroid carcinoma was carried out in variants using high-density oligonucleotide microarrays, and by quantitative RT-QPCR. Genes specific for papillary thyroid carcinoma were selected among genes, which expression differentiated PTC from normal thyroid tissue both in the full thickness and microdissected samples.

In the example described below, new tests based on gene expression and modifications were introduced to standard diagnostic methods for the identification of genes to increase the prediction of a diagnosis of thyroid nodule.

### EXAMPLE

### Sample preparation

Training set to select genes for expression classifier was chosen among more than 3,000 FNAB samples. The training set (1) consisted of 348 FNAB samples subjected to analysis of gene expression profile based on transcriptome (mRNA) assessment using microarray studies.

RNA isolation was performed using the miRNeasy Micro Kit according to the manufacturer's recommendations, whereas RNA concentration was measured by a high sensitivity kit using a fluorometer.

The RNA concentration obtained from 282 residual biopsy samples from the training set ranged between 4 ng/µl to 200 ng/µl, with a median of 16.7 ng/µl. However, for 160 samples the measurement results were below the sensitivity threshold (<20 ng/µl) of the device.

In the validation set RNA concentration from 164 samples ranged between 4 ng/µl to 184 ng/(µl and the median was 9.97 ng/µl. For 378 samples the measurement results were below the sensitivity threshold (<20 ng/µl) of the Qubit 2.0 device.

The microarray assay and quantitative PCR reaction preceded cDNA synthesis. The cDNA synthesis was performed on a RNA template. Random Nanomers and OligodT(23) primers and 40 units/µl of RNase inhibitor were also used. The initial RT buffer was diluted 3:1.

The reaction used 20 ng of the whole RNA for samples with measurable RNA concentration and 5.95 µl of RNA for samples with RNA concentration below the sensitivity threshold of the Qubit 2.0 device.

The composition of the cDNA synthesis is summarized in the table below:

| Reagents | Volume (per reaction) | Final concentration |
|---|---|---|
| 10x Buffer RT | 1 µl | 1x |
| dNTPs (5nM each) | 1 µl | 0,5 nM each |
| Random Nanomer Primers (50 µM) | 0,8 µl | 2,5 µM |
| Primer OligodT(n23) (40 µM) | 0,25 µl | 1 µm |
| RNAse Inhibitor (10 units/µl) | 0,5 µl | 1 unit |
| Polimeraze RT-O (4 units/µl) | 0,5 µl | 0,2 unit |
| RNAse free H₂O | 5,95 µl | |
| Final volume per sample | 10 µl | |

For RNA samples with a low RNA concentration, an additional pre-amplification step is used, which then allows a real-time PCR reaction without introducing amplification bias.

The pre-amplification step was carried out using TaqMan PreAmp Master Mix and Taqman Preamp Pool gene-specific primer mix. The preamplification reaction was carried out using QuantStudio 12k Flex System in 96-well plates, under the following conditions:
- AmpliTaq Gold DNA polymerase activation - 10 minutes, 95°C
- Denaturation - 15 seconds, 95°C
- primer attachment and chain extension - 1 minute, 60°C.
- completion of reaction - 10 minutes, 99°C
The number of cycles varied, and it was:
- 14 cycles for samples with 20ng RNA concentration
- 18 cycles for samples with concentrations below the sensitivity threshold.

After the reaction, the sample was diluted 20x by adding 190 ul of RNase-free water.

The composition of the cDNA preamplification reaction is summarized in the table below:

| Reagents | Volume (per reaction) | Final concentration |
|---|---|---|
| 2x Taqman Preamp Master mix | 5 µl | 1x |
| Taqman Preamp Pool | 2,5 µl | |
| cDNA template | 2,5 µl | |
| Final volume | 10 µl | |

The length of the obtained amplicons ranged between 53 base pairs and 134 base pairs (see table 1; amplicon length).

In the next step, the reaction mixture, RNase-free water, and preamplified cDNA were applied to a 384-well plate. The plate was centrifuged for 1 min at 2000 rpm. Next, the samples were then transferred from the 384-well plate to the OpenArray plate in a dispensing station. The OpenArray plate was sealed with a lid and placed in the press for 10s. After removal, the plate was filled with oil and sealed with a cap.

The composition of the cDNA amplification reaction is summarized in the table below.

| Reagents | Volume (per reaction) |
|---|---|
| 2x Taqman Openarray Real-Time PCR Mastermix | 2,6 µl |
| RNAse free H₂O | 1,4 µl |
| cDNA template (preamplified) | 1,5 µl |
| Final volume | 5,5 µl |

The QPCR reaction was performed using QuantStudio 12K FLEX System with an OpenArray block according to the OpenArray Taqman Gene Expression protocol.

The gene expression profiling can be performed either by the RT-QPCR presented above or by oligonucleotide microarrays or transcriptome sequencing (RNAseq; next-generation sequencing (NGS).

In addition, the above-mentioned processes can also be used to amplify genes in a frozen sample or fresh-frozen biopsy material or fixed paraffin blocks containing RNA at concentrations ranging from 4 ng/µl to 20 ng/µl.

### Normalization

The selection of reference genes is important to carry out proper validation of trait selection. The analysis of gene expression variability was conducted using a set of microarray data included in the Training set. The procedure aims to select those genes whose variability is the smallest between all the samples studied. For the analysis of the samples, gene stability was also confirmed using RT-QPCR.

For example, for a reliable classification quality for the OpenArray platform, the values of AplificationScore parameter <1.1 and a cut-off cycle (Ct) value less than 40 (for good amplification < 28) are accepted.

The essential issue of this analysis was to evaluate not only single genes as candidates for reference genes but also groups of genes able to perform these functions together. Due to the importance of the correctness of the result - the normalization process is carried out using normalizing genes, characterized by stable expression. For normalization, each plate contains calibrator samples.

The normalizing genes determined by feature selection are EIF3A (EIF3S10), HADHA, UBE2D2, EIF5, PPIE, RPLP0.

The calibrator samples are the RNA used to normalize the data between runs of the analysis (subsequent plates). It is intended that each sample should show good expression for at least four reference genes.

The normalization result is subject to analysis, including verification that all reference genes return a signal from the exemplary referenced quantitative RT-qPCR reaction. The normalized gene expressions and cytological score are entered into the classifier using a .csv or .xlsx file. Based on the gene expression profile analysis and using the above-mentioned normalizing genes, a gene set of the expression classifier is established.

In the first classifier set, the optimal gene list includes the following 20 genes C3, CAMK2N1, DUSP5, EGR1, EMP2, FAM20A, FAXC, ITGA2, KCNQ3, MET, METTL7B, MPZL2, PSD3, SDC4, SLC26A4, SLPI, TM4SF1, TPO, SLC26A7, and TUSC3 (Table 1).

As already described, the classifier retrieves patient gene expression values measured by RT-QPCR through the BioMaps application interface, performs classification using the model SVM. Displays the classification result on a computer screen as a boxplot (possibly a mandolin with dots, or histograms) for the benign and malignant class of each gene with the expression value of the test sample and shows the class prediction, the malignancy probability, and the numerical values of all the features of the sample on the basis of which classification was done.

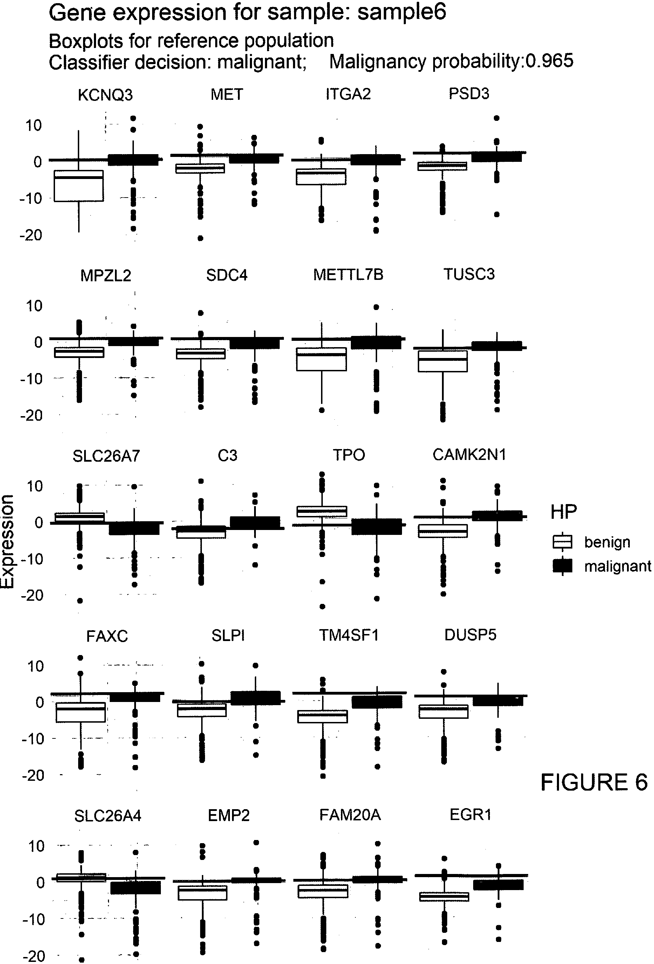

An example application of a 20-gene classifier to analyze gene expression and determine a predictive value is shown in the box plot in figure 6. The result predicted by the classifier indicates a malignant type of the tumor sample (the probability of malignancy 0.965).

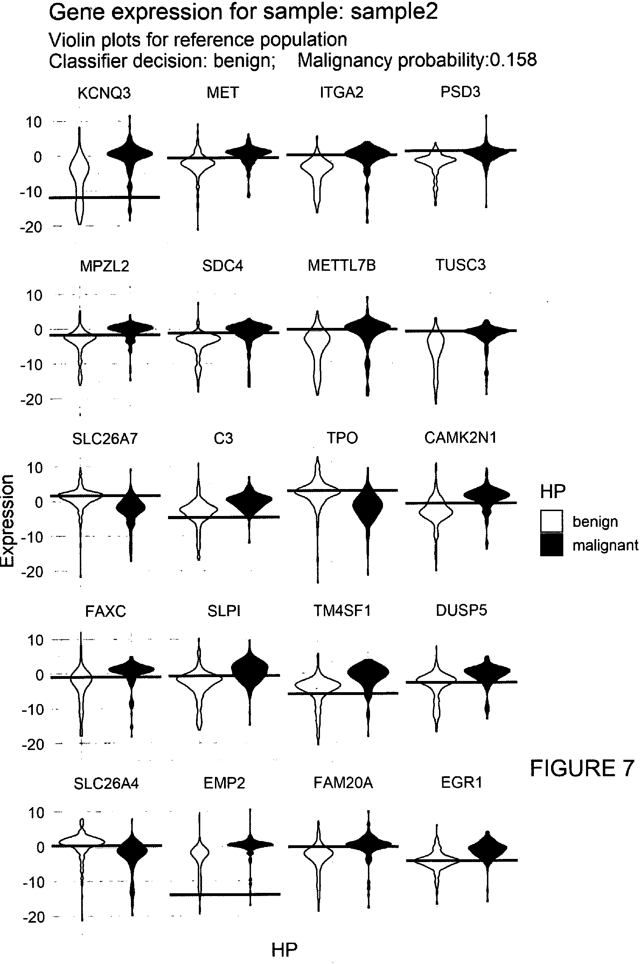

Other exemplary application of the 20 gene classifier to analyze gene expression and determining the predictive value is shown in the mandolin diagram in figure 7. The result predicted by the classifier indicates a benign type of tumor sample (the probability of malignancy 0.158).

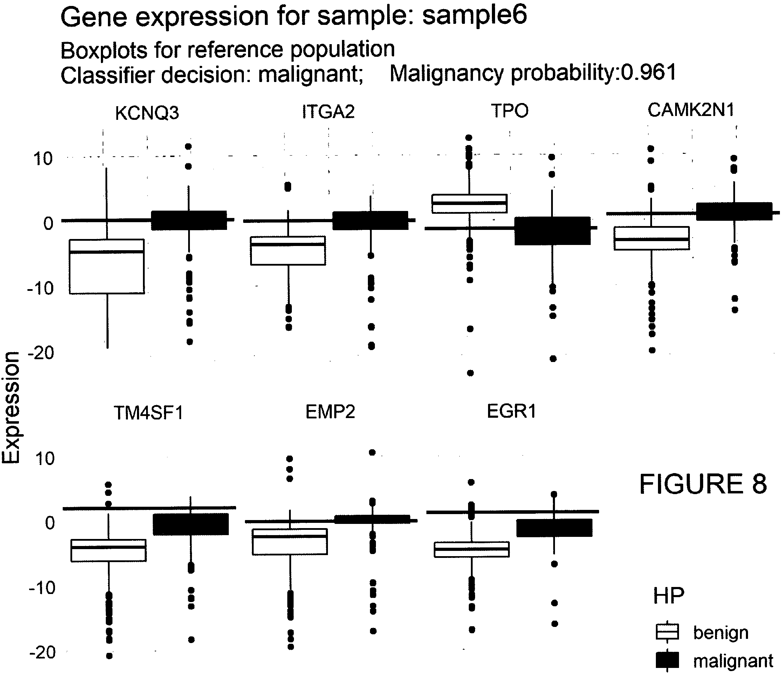

An example application of the 7 -gene classifier to analyze gene expression and determine the predictive value is shown in the box plot in figure 8. The result predicted by the classifier indicates a malignant type of the tumor sample (the probability of malignancy 0.961).

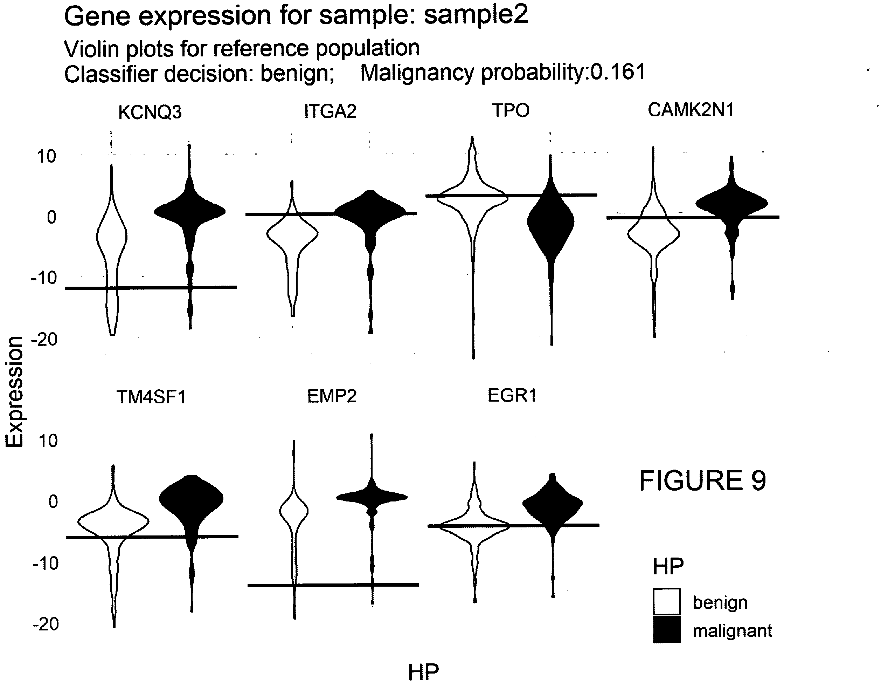

Other exemplary application of the 7-gene classifier to analyze gene expression and determine the predictive value is shown in the mandolin diagram in figure 9. The result predicted by the classifier indicates a benign type of the tumor sample, (probability of malignancy 0.161).

### Referrences:

1.Alexander, Erik K, Giulia C Kennedy, Zubair W Baloch, Edmund S Cibas, Darya Chudova, James Diggans, Lyssa Friedman, et al. 2012. "Preoperative Diagnosis of Benign Thyroid Nodules with Indeterminate Cytology." The New England Journal of Medicine 367 (8): 705-15. https://doi.org/10.1056/NEJMoa1203208.
2.Chudova, Darya, Jonathan I Wilde, Eric T Wang, Hui Wang, Nusrat Rabbee, Camila M Egidio, Jessica Reynolds, et al. 2010. "Molecular Classification of Thyroid Nodules Using High-Dimensionality Genomic Data." The Journal of Clinical Endocrinology and Metabolism 95 (12): 5296-5304. https://doi.org/10.1210/jc.2010-1087.
2.González. Hernán E., José R. Martinez, Sergio Vargas-Salas, Antonieta Solar, Loreto Veliz, Francisco Cruz, Tatiana Arias, et al. 2017. "A 10-Gene Classifier for Indeterminate Thyroid Nodules: Development and Multicenter Accuracy Study." Thyroid 27 (8): 1058-67. https://doi-org/10.1089/thy.2017.0067.
4.Lithwick-Yanai, Gila, Nir Dromi, Alexander Shtabsky, Sara Morgenstern, Yulia Strenov, Meora Fein-messer, Vladimir Kravtsov, et al. 2017. "Multicentre Validation of a MicroRNA-Based Assay for Diagnos-ing Indeterminate Thyroid Nodules Utilising Fine Needle Aspirate Smears." Journal of Clinical Pathology 70 (6): 500-507. https://doi.org/10.1136/jclinpath-2016-204089.
5.Nikiforov, Yuri E., Sally E. Carty, Simon I. Chiosea, Christopher Coyne, Umamaheswar Duvvuri, Robert L. Ferris, William E. Gooding, et al, 2015. "Impact of the Multi-Gene ThyroSeq Next-Generation Sequencing Assay on Cancer Diagnosis in Thyroid Nodules with Atypia of Undetermined Significance/Follicular Le-sion of Undetermined Significance Cytology." Thyroid 25 (11): 1217-23. https://doi.org/10.1089/thy.2015.0305.
6.Zafereo, Mark, Bryan McIver, Sergio Vargas-Salas, Jose Miguel Domínguez, David L. Steward, F. Chris-topher Holsinger, Emad Kandil, et al. 2020. "A Thyroid Genetic Classifier Correctly Predicts Benign No-dules with Indeterminate Cytology: Two Independent, Multicenter, Prospective Validation Trials." Thyro-id 30 (5): 704-12. https://doi.org/10.1089/thy.2019.0490.
7.Haugen B, Alexander E, Bible KC, Doherty GM, Mandel SJ,.Nikiforov YE, Pacini F I wsp. 2015 American Thyroid Association Management Guidelines for Adult Patients with Thyroid Nodules and Differentiated Thyroid Cancer: The American Thyroid Association Guidelines Task Force on Thyroid Nodules and Diffe-rentiated Thyroid Cancer. Thyroid. 2016 Jan 1; 26(1): 1-133
8.Cibas ES, Ali SZ. The 2017 Bethesda System for Reporting Thyroid Cytopathology. Thyroid. 2017 Nov;27(11): 1341-1346

## Claims

1. A method of testing genes for diagnosing thyroid nodules in a sample from biopsy (FNAB) material, from which RNA is isolated, then RNA upon transcription is synthesized into cDNA, followed by determination and normalization of gene expression, **characterized in that**:
- the material is a frozen or freshly collected residual biopsy, obtained by washing the residue in the bevel of the needle, containing RNA at concentrations ranged between 4 ng/µl and 20 ng/µl, which is pre-amplified before transcription of total RNA at a number of cycles depending on RNA concentration, whereas the length of the obtained gene amplicons ranges between 53base pairs and 134 base pairs,
- the expression of genes selected from transcription factors of the corresponding target gene is analyzed,
- the normalization of expression of target genes is carried out under endogenous control of at least four normalizing genes, preferably six normalizing genes, whose gene amplicon lengths are in the range of 50 base pairs - 140 base pairs.

2. The method, according to claim # 1, is **characterized in that** the analyzed material is a frozen or freshly collected biopsy sample containing RNA at a concentration ranged from 4 ng/µl to 20 ng/µl.

3. The method, according to claim # 1 or 2, is **characterized in that** the expression profile of a set of the following genes C3, CAMK2N1, DUSP5, EGR1, EMP2, FAM20A, FAXC, ITGA2, KCNQ3, MET, METTL7B, MPZL2, PSD3, SDC4, SLC26A4, SLPI, TM4SF1, TPO, SLC26A7, and TUSC3 is determined; and combined expression levels above certain threshold point a malignant character of the nodule.

4. The method, according to claim # 1 or 2, is **characterized in that** the expression profile of at least seven genes of the set comprising C3, CAMK2N1, DUSP5, EGR1, EMP2, FAM20A, FAXC, ITGA2, KCNQ3, MET, METTL7B, MPZL2, PSD3, SDC4, SLC26A4, SLPI, TM4SF1, TPO, SLC26A7, and TUSC3 is determined; and combined expression levels above certain threshold point a malignant character of the nodule.

5. The method, according to claim # 4, is **characterized in that** the expression profile of 7 genes, including CAMK2N1, EGR1, EMP2, ITGA2, KCNQ3, TM4SF1, and TPO, is determined.

6. A kit for diagnosis of thyroid nodules in a biopsy specimen, comprising a device with at least one processor and at least one program memory, which contains the processor's execution instructions for data analysis and determination of gene expression profile by the algorithm of the classifier, and furthermore having devices, as well as suitable reagents, solutions, probes and primers for conducting reverse transcription reactions, and devices with equipment for detecting the expression of a set of 20 genes or at least 7 genes from a set comprising C3, CAMK2N1, DUSP5, EGR1, EMP2, FAM20A, FAXC, ITGA2, KCNQ3, MET, METTL7B, MPZL2, PSD3, SDC4, SLC26A4, SLPI, TM4SF1, TPO, SLC26A7, and TUSC3.

7. A kit, according to claim # 6 is **characterized in that** the set of 7 genes includes CAMK2N1, EGR1, EMP2, ITGA2, KCNQ3, TM4SF1, and TPO.

8. A kit, according to claim # 6 is **characterized in that** devices and equipment used in the method for gene expression analysis involve RT-qPCR, RNAseq oligonucleotide microarrays, or NGS transcriptome sequencing.

9. The use of genes from the set including C3, CAMK2N1, DUSP5, EGR1, EMP2, FAM20A, FAXC, ITGA2, KCNQ3, MET, METTL7B, MPZL2, PSD3, SDC4, SLC26A4, SLPI, TM4SF1, TPO, SLC26A7, and TUSC3, for diagnosing thyroid nodules in an ultra-small residual biopsy sample or full biopsy with RNA concentrations ranging from 4 ng/µl to 20 ng/µl.

10. The use of genes from the set including CAMK2N1, EGR1, EMP2, ITGA2, KCNQ3, TM4SF1, and TPO for diagnosing thyroid nodules in an ultra-small residual biopsy or full biopsy with RNA concentrations ranging from 4 ng/µl to 20 ng/µl.
